# EUROPEAN PATENT SPECIFICATION

(11) **EP 2 825 259 B1**
(45) Date of publication and mention of the grant of the patent: **07.06.2017**
(21) Application number: 13714087.7
(22) Date of filing: 13.03.2013
(51) Int. Cl.: A61N 5/06, A61N 5/10

(54) **SYSTEM FOR FACILITATING PHOTOTHERAPEUTIC TREATMENT**
SYSTEM FÜR LICHTTHERAPIEBEHANDLUNG
SYSTÈME DESTINÉ À FACILITER UN TRAITEMENT PHOTOTHÉRAPEUTIQUE

(30) Priority: 13.03.2012 US 201261610052 P
(43) Date of publication of application: 21.01.2015
(73) Proprietor: National Biological Corporation, Beachwood, OH 44122 (US)
(72) Inventor: KAUFMAN, Michael, Beachwood, OH 44122 (US)
(74) Representative: Barton, Matthew Thomas
(86) International application number: PCT/US2013/031034
(87) International publication number: WO 2013/138517

(56) References cited:
- WO-A1-02/075612
- WO-A1-2011/038310
- US-A1- 2008 069 303

## Description

### BACKGROUND

Phototherapy relates to the treatment of disease, such as skin conditions including psoriasis, acne, eczema, vitiligo, and mycosis fungoides, by exposure to light, especially by various concentrated light rays such as long-wave or short-wave ultraviolet light. The treatment may be alone or in combination with a sensitizer which sensitizes the skin to light.

In particular, ultraviolet light ("UV light," radiation in the region of the electromagnetic spectrum including wavelengths from 280 to 400 nanometers) has been used for over 30 years in a clinical setting for treating severe skin diseases, such as psoriasis and vitiligo. This treatment regimen is called UV phototherapy. The UV light spectrum is either UVA or UVB and the treatment course is typically three times a week for three months. Treatments can take place in a clinical location or in a home setting. Administration of existing phototherapy systems, however, is not centralized. Thus, the treatments may not be optimally administered. WO 2011/038310 A1 teaches a system not incorporating a treatment rule as defined in claim 1.

### SUMMARY

The invention is defined in independent claim 1. A phototherapeutic device comprises a system for facilitating phototherapeutic treatment. The system comprises an input device configured to receive input from a user. The system further comprises a display device configured to communicate information to the user. The system further comprises a controller. The controller is configured to receive user identification data from the input device. The controller is further configured to retrieve treatment history based on the user identification data. The controller is further configured to retrieve a treatment protocol based on the user identification data. The controller is further configured to generate a suggested treatment based on the retrieved treatment history and the retrieved treatment protocol. The controller is further configured to communicate the suggested treatment to the display device.

A system for facilitating medical treatment comprises at least one processor, at least one computer-readable tangible storage device, and program instructions stored on the at least one storage device for execution by the at least one processor. The program instructions comprise first program instructions configured to receive identification data representative of a patient. The program instructions further comprise second program instructions configured to retrieve treatment history based on the identification data. The program instructions further comprise third program instructions configured to retrieve a treatment protocol based on the identification data. The program instructions further comprise fourth program instructions configured to generate a suggested treatment based on the retrieved treatment history and the retrieved treatment protocol. The program instructions further comprise fifth program instructions configured to communicate the suggested treatment to the patient.

A method for facilitating phototherapeutic treatment comprises the step of a computer receiving identification data representative of a patient. The method further comprises the step of a computer retrieving treatment history based on the identification data. The method further comprises the step of a computer retrieving a treatment protocol based on the identification data. The method further comprises the step of a computer generating a suggested treatment based on the retrieved treatment history and the retrieved treatment protocol. The method further comprises the step of a computer communicating the suggested treatment.

### BRIEF DESCRIPTION OF THE DRAWINGS

The accompanying drawings, structures are illustrated that, together with the detailed description provided below, describe exemplary embodiments of the claimed invention. Like elements are identified with the same reference numerals. It should be understood that elements shown as a single component may be replaced with multiple components, and elements shown as multiple components may be replaced with a single component. The drawings are not to scale and the proportion of certain elements may be exaggerated for the purpose of illustration.
**FIG. 1** illustrates a phototherapeutic system comprising a plurality of networked phototherapeutic devices in accordance with one non-limiting embodiment.
**FIGS. 2A-2D** illustrate phototherapeutic devices in accordance with non-limiting embodiments.
**FIG. 3** illustrates a block diagram of an example phototherapeutic controller in accordance with non-limiting embodiments.
**FIG. 4** illustrates a phototherapeutic system comprising a plurality of networked ancillary phototherapeutic devices in accordance with one non-limiting embodiment.
**FIG. 5** illustrates a phototherapeutic system comprising a plurality of networked ancillary phototherapeutic devices and a full-body phototherapeutic device in accordance with one non-limiting embodiment.
**FIGS. 6A-6B** are diagrams of example user interfaces of a display device in accordance with one non-limiting embodiment.
**FIG. 7** is a flow chart illustrating the steps of an example method for phototherapeutic treatment.

### DETAILED DESCRIPTION

Various non-limiting embodiments of the present disclosure will now be described to provide an overall understanding of the principles of the structure, function, and use of the phototherapeutic systems and processes disclosed herein. One or more examples of these non-limiting embodiments are illustrated in the accompanying drawings. Those of ordinary skill in the art will understand that systems and methods specifically described herein and illustrated in the accompanying drawings are non-limiting embodiments. The features illustrated or described in connection with one non-limiting embodiment may be combined with the features of other non-limiting embodiments. Such modifications and variations are intended to be included within the scope of the present disclosure.

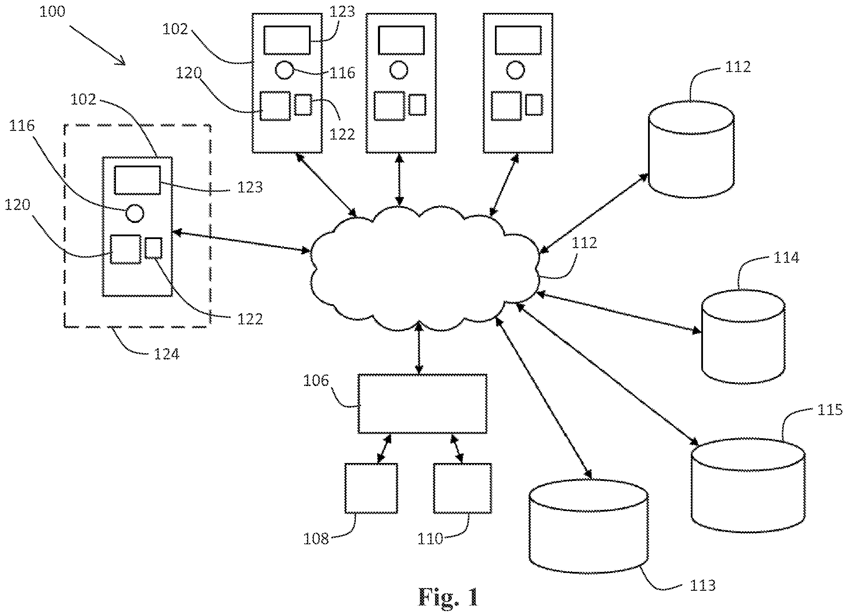

**FIG. 1** illustrates a phototherapeutic system 100 comprising a plurality of networked phototherapeutic devices 102 in accordance with one non-limiting embodiment. Phototherapeutic devices 102 may be in communication with a communications network. The communications network may include a number of computer and/or data networks, including the Internet, LANs, WANs, GPRS networks, etc., and may comprise wired and/or wireless communication links. Furthermore, phototherapeutic devices 102 may each be in communication with communications network via any suitable type of suitable network connection, including wireless connections, wired connections, and hybrid wired-wireless networks. For example, phototherapeutic devices 102 may be in communication with the communications network via WiFi, ZigBee, Bluetooth, Ethernet, telephone line, and the like.

It should be understood that, although the example systems and methods described herein make reference to phototherapeutic devices and phototherapeutic treatments, the example systems and methods may also be used with other suitable medical devices for other suitable medical treatments.

A phototherapeutic controller 106 may be in communication with communications network. An input device 108 may be used for interacting with phototherapeutic controller 106. Input device 108 may be, for example, a keyboard, a stylus, a touch-sensitive screen, keys (e.g., input keys, preset and programmable hot keys), buttons (e.g., action buttons, a multidirectional navigation button, preset and programmable shortcut buttons), and so forth. A display device 110 may also be in communication with phototherapeutic controller 106.

Phototherapeutic controller 106 may be configured to interact with and control phototherapeutic devices 102. Accordingly, display device 110 may visually present information to an operator of phototherapeutic device 102 or to a patient receiving phototherapeutic treatment from phototherapeutic device 102. Display device may also provide an interface to enable an operator to initiate and terminate a treatment process, via phototherapeutic device 102, either locally or from a remote location. In some embodiments, information such as patient information and equipment status information may be presented in a real time graphical format.

Phototherapeutic system 100 may further comprise various data structures for storing information related to the phototherapeutic system 100. In one embodiment, as illustrated, a patient database 112 is used to store information related to patients and an equipment database 114 is used to store information related to phototherapeutic devices 102. Patient database 112 may be administered internally to Phototherapeutic system 100 by the administrator of phototherapeutic controller 106 or patient database 112 may be an external third party administered database. For example, patient database 112 may be an electronic medical records (EMR) database. Data retrieved from and EMR database may include patient ID, physician information, insurance company information, and past medical history. Similarly, treatment data such as date, time, UV light exposure level, diagnosis, and procedure may be stored on the EMR database as well.

Phototherapeutic system 100 may further comprise additional databases, such as a medical professionals database 113 and a treatment protocol database 115. The medical professionals database 113 may store data related interaction with phototherapeutic devices 102 by medical professionals. For example, the database may store information related to times/dates of when a nurse logged into a particular phototherapeutic device 102 and/or logged into phototherapeutic controller 106. Treatment protocol database 115 may store data relating to phototherapeutic treatments according to various protocols. For example, information related to treatment settings, parameters, and so forth may be stored in this database. Additional suitable data may be stored in the databases associated with phototherapeutic system 100, such as patient exposure history, patient biometric data, equipment historical use data, and so forth.

In accordance with various embodiments, phototherapeutic devices 102 may have a variety of additional features, controls, and so forth. For example, a call device 116 may be coupled to phototherapeutic device 102. The call device 116 may be any suitable device, such as a call button, push button, pull chain, touch-sensitive plate, push bar, and so forth. During treatment by phototherapeutic device 102, a patient may request to call the nurse, or other assistant. Accordingly, activation of call device 116 may trigger a notice on display device 110. In some embodiments, phototherapeutic controller 106 may be configured to electronically transmit a notice in response to activation of call device 116. Phototherapeutic controller 106 may coordinate the dispatch of a cell phone text message, an email message, a phone call, an instant message, or other type of electronic notification to a nurse, an operator, or other suitable recipient.

Phototherapeutic devices 102 may also include an input device 120. Input device 120 may be used by a patient to input information, such as patient identification information. Input device 120 may be a biometric input device (e.g., for fingerprint scanning, retina scanning, etc.), a keypad (i.e., for entering a unique patient identification number), a touchscreen, or other suitable input device. Based on the patient identification information supplied, phototherapeutic controller 106 may query patient database 112 to retrieve treatment records, patient data, and so forth. Phototherapeutic controller 106 may use the retrieved data as a guideline for recommending current treatment at phototherapeutic devices 102. In one example, input device 120 may be used to adjust the suggested treatment. For example, a patient may wish to increase or decrease the suggested exposure. In one example, phototherapeutic controller 106 may prevent a patient from adjusting the suggested treatment without proper permission. For example, phototherapeutic controller 106 may require an administrator with appropriate access credentials to adjust the suggested treatment.

In one example, phototherapeutic controller 106 may also retrieve a protocol for administering treatment, from treatment protocol database 115, based on the retrieved data. For example, treatment protocol database 115 may include predefined rules that specify initial treatment protocols depending on skin type and shade. Accordingly, phototherapeutic controller 106 may be configured to recommend a first treatment protocol for an identified patient having a first skin type and be configured to recommend a second treatment protocol for an identified patient having a second skin type. In addition, treatment protocol database 115 may define rules that specify what percentage to increase exposure between treatments, or what percentage to decrease exposure if a treatment was missed. In some embodiments, phototherapeutic controller 106 may be configured to cause phototherapeutic device 102 to perform a treatment and to monitor progress of the treatment.

In some embodiments, phototherapeutic devices 102 may have an annunciator 122 for announcing the name of the patient. Annunciator 122 may be, for example, a speaker to provide an audio announcement of the patient's name or a display to provide a visual announcement of the patient's name (or other patient identifier). Phototherapeutic devices 102 may also include a display device 123 for conveying various types of information to a nurse, a patient, or a technician, for example. In one embodiment, once a patient provides their identity (via biometrics or otherwise), the patient's name and treatment protocol may be displayed on display device 123. Display device 123 may also display a digital photograph of the patient to aid in the proper identification of the patient prior to treatment. A calendar may be displayed on display device 123 that indicates past treatments for that patient. For example, the calendar may indicate on which days the patient has received the treatment. Given the repetitive nature of phototherapy treatment regiment, exposure history typically provides a guideline for recommending the parameters of the current treatment's exposure. Displaying past treatments in a calendar may assist in visualizing exposure trends. In addition to displaying past treatments in a calendar form, display device 123 may also display a suggested treatment schedule for future treatments in a calendar form.

Display device 123 may also be configured to display progress of a current treatment being administered. For example, display device 123 may display a progress or status bar to provide a real-time visual display of the current treatment status.

Although **FIG. 1** depicts a limited number of elements for purposes of illustration, it can be appreciated that phototherapeutic system 100 may include more or less elements as well as other types of elements in accordance with the described embodiments. Elements of phototherapeutic system 100 may include physical or logical entities for communicating information implemented as hardware components (e.g., computing devices, processors, logic devices), executable computer program instructions (e.g., firmware, software) to be executed by various hardware components, or combination thereof, as desired for a given set of design parameters or performance constraints. Furthermore, phototherapeutic devices 102 may be located in the same environment (i.e., a clinical setting), or may be located remote from each other, as indicated by remote location 124. Moreover, phototherapeutic controller 106 may be in a location remote from one or more associated phototherapeutic devices 102. For example, a plurality of clinical locations can each be networked to a central phototherapeutic controller 106 through communications network 104. Furthermore, display device 123 may located at phototherapeutic device 102, at phototherapeutic controller 106, or at a third location, remote from both phototherapeutic device 102 and phototherapeutic controller 106. Thus, in one example, phototherapeutic controller 106 may include a transceiver for communicating with multiple phototherapeutic devices 102 over a wired or wireless network.

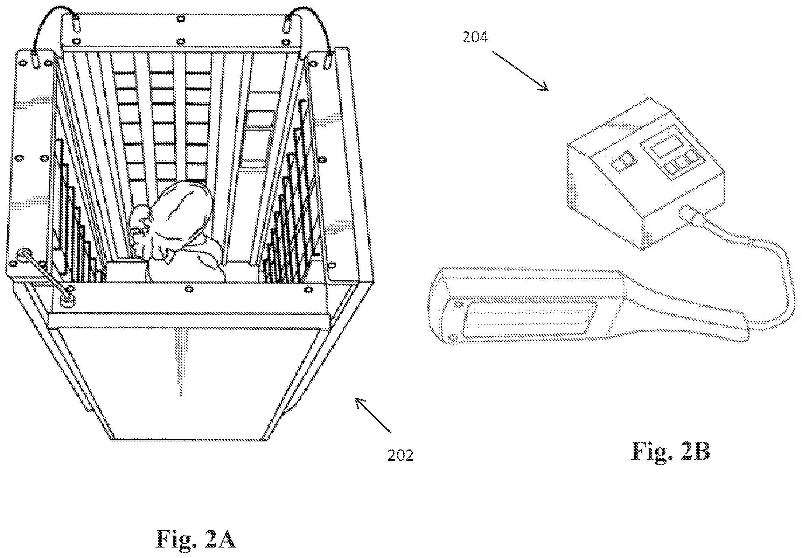

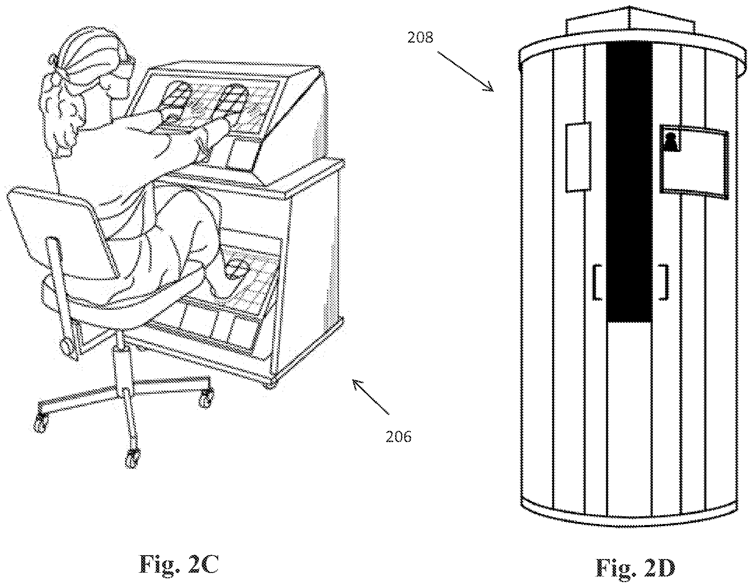

Phototherapeutic devices 102 depicted in **FIG. 1** are illustrated as 6-foot (183 cm) tall panels. However, the system and method described herein are not so limited. For example, a phototherapeutic device 102 could be in the form of a tanning bed or a booth that surrounds the patient. The device could also be, for example, a 2-foot (61 cm) panel, a handheld wand, or a desk-type device for the treatment of hands and/or feet. An exemplary folding booth 202 is shown in **FIG. 2A****,** an exemplary handheld wand 204 is shown in **FIG. 2B****,** an exemplary desk-type device 206 is shown in **FIG. 2C** for both hands and feet, and an exemplary non-folding booth 208 is shown in **FIG. 2D****,** each of which may be used in accordance with the presently disclosed system and method. Each of these devices may be networked to phototherapeutic controller 106 via communications network 104 via a network connection and have one or more of the features illustrated in **FIG. 1****,** such as call device 116, input device 120, annunciator 122, and display device 123.

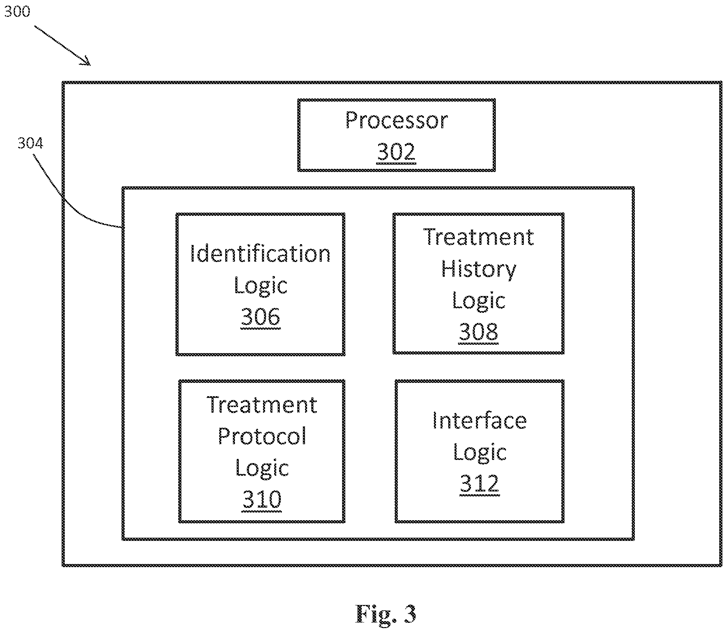

**FIG. 3** illustrates a block diagram of an example phototherapeutic controller 300 in accordance with non-limiting embodiments. Phototherapeutic controller 300 may be provided using any suitable processor-based device or system, such as a personal computer, laptop, server, mainframe, or a collection (e.g., network) of multiple computers, for example. Phototherapeutic controller 300 may include one or more processors 302 and one or more computer memory units 304. For convenience, only one processor 302 and only one memory unit 304 are shown in **FIG. 3****.**

Memory unit 304 includes logic or program instructions configured to perform specific tasks. Particularly, memory unit 304 includes identification logic 306 configured to receive identification data representative of a patient. Memory unit 304 further includes treatment history logic 308 configured to retrieve treatment history based on the identification data. Memory unit 304 further includes treatment protocol logic 310 configured to retrieve a treatment protocol based on the identification data. Memory unit 304 further includes treatment suggestion logic 312 configured to generate a suggested treatment based on the retrieved treatment history and the retrieved treatment protocol. Memory unit 304 further includes interface logic 312 configured to communicate the suggested treatment to the patient. In one example, interface logic is further configured to initiate a phototherapeutic device to perform the suggested treatment, responsive to receiving confirmation from a user to begin treatment.

Processor 302 may execute software instructions 306-312 stored on memory unit 304. Processor 302 may be implemented as an integrated circuit (IC) having one or multiple cores. Memory unit 304 may include volatile and/or non-volatile memory units. Volatile memory units may include random access memory (RAM), for example. Non-volatile memory units may include read only memory (ROM), for example, as well as mechanical non-volatile memory systems, such as, for example, a hard disk drive, an optical disk drive, etc. The RAM and/or ROM memory units may be implemented as discrete memory ICs, for example. Data used by phototherapeutic controller 300 may be from various sources, such as patient database 112 or equipment database 114 of **FIG. 1****,** which may be an electronic computer database, for example.

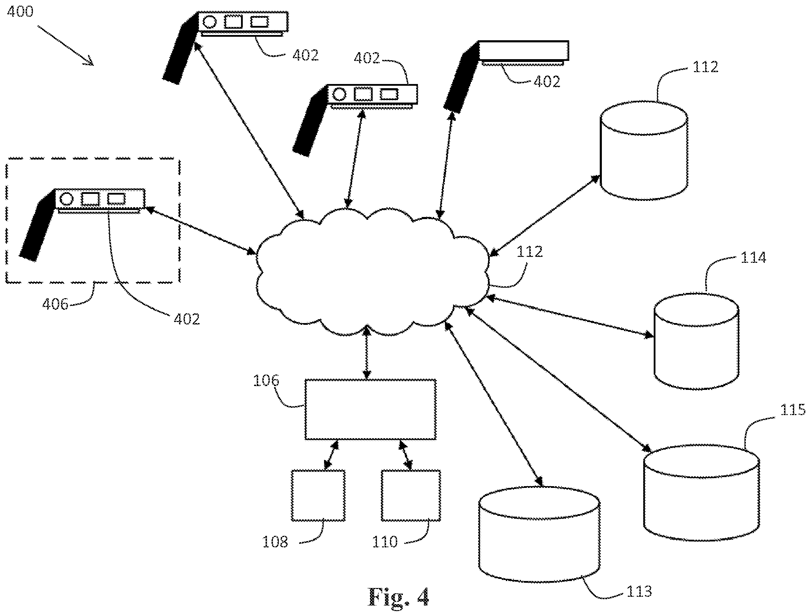

**FIG. 4** illustrates a phototherapeutic system 400 comprising a plurality of networked ancillary phototherapeutic devices 402 in accordance with one non-limiting embodiment. In the illustrated embodiment, phototherapeutic devices 402 are handheld devices which may be used in a clinical setting or a home-setting. As such, one or more phototherapeutic devices 402 may be in a location remote from phototherapeutic controller 106, as indicated by remote location 406. Furthermore, it is to be understood that while four phototherapeutic devices 402 are illustrated in **FIG. 4****,** any number of phototherapeutic devices 402 may be in communication with the communications network 104. One or more phototherapeutic devices 402 may have features similar to call device 116, input device 120, annunciator 122, and display device 123, as described above with reference to **FIG. 1****.**

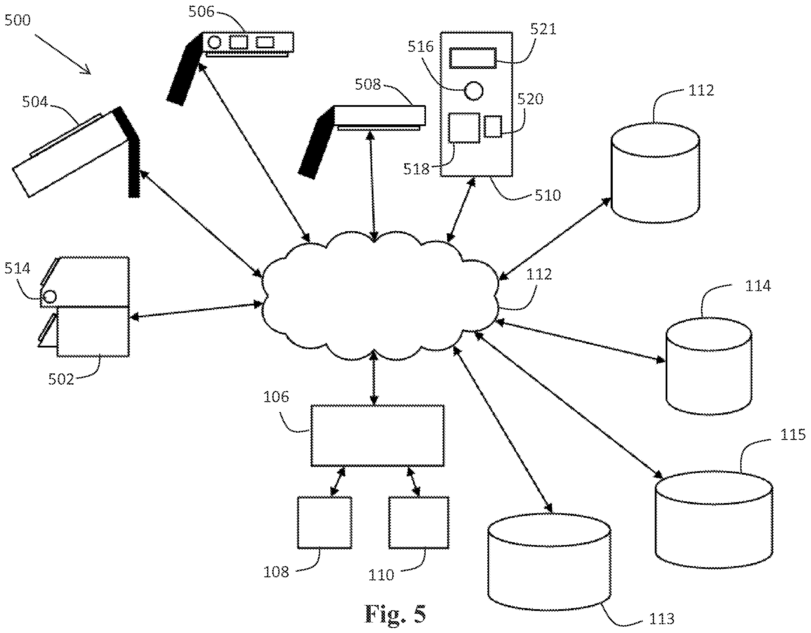

A variety of phototherapeutic device types may be in communication with a phototherapeutic controller 106. **FIG. 5** illustrates a phototherapeutic system 500 comprising a plurality of networked ancillary phototherapeutic devices and a full-body phototherapeutic device in accordance with one non-limiting embodiment. In the illustrated embodiment, the phototherapeutic device types associated with the system include a desk-type phototherapeutic device 502, a 2-foot (61 cm) panel phototherapeutic device 504, a handheld phototherapeutic device 506, a handheld phototherapeutic device 508, and a booth-type phototherapeutic device 510. Each of the devices may have one or more control features similar to call device 116, input device 120, annunciator 122, and display device 123, as described above with reference to FIG. 1. For example , desk-type phototherapeutic device 502 has a call device 514 while booth-type phototherapeutic device 510 has a call device 516, an input device 518, an annunciator 520, and a display 521.

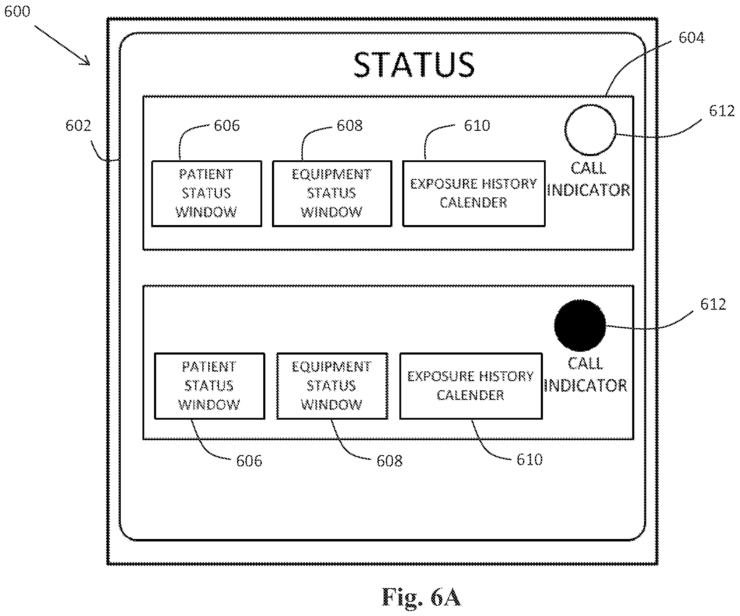

**FIG. 6A** shows an example embodiment of a user interface 600 for interfacing with phototherapeutic controller 106 of **FIG. 1****.** User interface 600 may convey a wide variety of information to the user and may enable a user to communicate and interact with phototherapeutic controller 106. User interface 600 may be displayed on display device 110 of **FIG. 1****.** It is to be appreciated, that the present disclosure is not limited to the arrangement and content of user interface 600 illustrated in **FIG. 6A****.** User interface 600 may comprise a status window 602. In the illustrated embodiments, status window 602 may comprise phototherapeutic device status windows 604. Phototherapeutic device status windows 604 may be associated with phototherapeutic devices 102 coupled to the phototherapeutic system 100.

Phototherapeutic device status windows 604 may visually present data or information that is stored in patient database 112, equipment database 114, medical professionals database 113, and treatment protocol database 115. The information may be presented in real time graphical format. In one embodiment, the phototherapeutic device status windows 604 comprise a patient status window 606, an equipment status window 608, and an exposure history calendar 610. Exposure history calendar 610 may present, for example, a rolling four week calendar graphically displaying the exposure history and exposure trends of the patient using the associated phototherapeutic device 102. Interface 600 may also comprise a call indicator display 612. Call indicator display 612 in a phototherapeutic device status windows 604 changes color or appearance when a patient activates a call button in an associated phototherapeutic device 102.

As is to be appreciated, user interface 600 may display a variety of useful information to a user. Such information may include, without limitations, patient name, current exposure duration, and so forth.

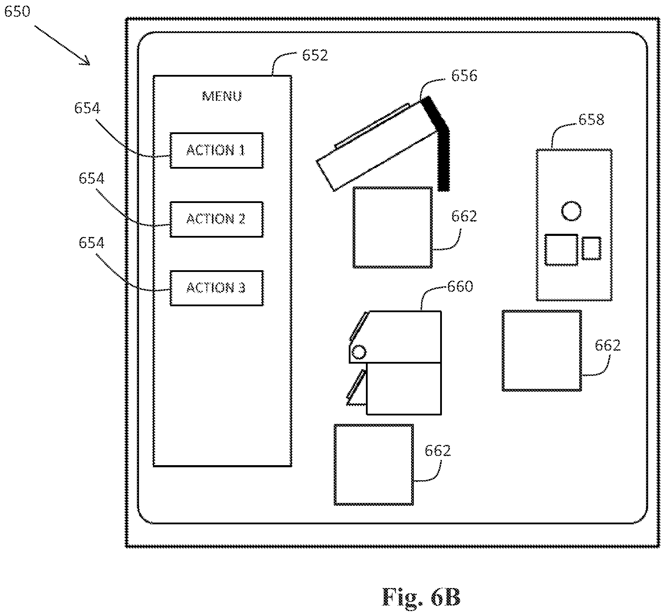

**FIG. 6B** shows another example embodiment of a user interface 650 which provides a dashboard to convey a wide variety of information to the user. User interface 650 may be displayed on display device 110 of **FIG. 1****.** It is to be appreciated, that the present disclosure is not limited to the arrangement and content of user interface 650 illustrated in FIG. 6B. User interface 650 may comprise a menu field 652. Menu field 652 may contain one or more action buttons 654. Activation of an action button 654 by an operator may trigger phototherapeutic controller 106 to perform a particular function or activity, such as accessing treatment protocols, billing information, patient databases, and so forth.

User interface 650 may also pictorially show the associated phototherapeutic devices 102, illustrated as device image 656, 658, and 660. Status information may be displayed in device status windows 662, 664, 666, positioned proximate each device image 656, 658, and 660, respectively. The device status windows 662, 664, 666 may indicate, for example, a nurse name, a patient name, a device status, and a time (duration) of treatment. In some embodiments, user interface 650 may be displayed on a touch sensitive screen (such as a desktop computer, tablet, smartphone, or laptop, for example). A nurse, or other user, can touch the screen proximate the device image 656, 658, 660 to display a screen with additional information. As is to be appreciated, user interface 650 may display a variety of useful information to a user. Such information may include, without limitations, patient name, current exposure duration, and so forth.

It should be appreciated that user interfaces 600 and 650 may be configured to display information intended for a patient, such as treatment information, as well as information intended for an administrator, such as systems status information. Accordingly, information displayed and functionality made available by user interfaces 600 and 650 may change depending on the user identified.

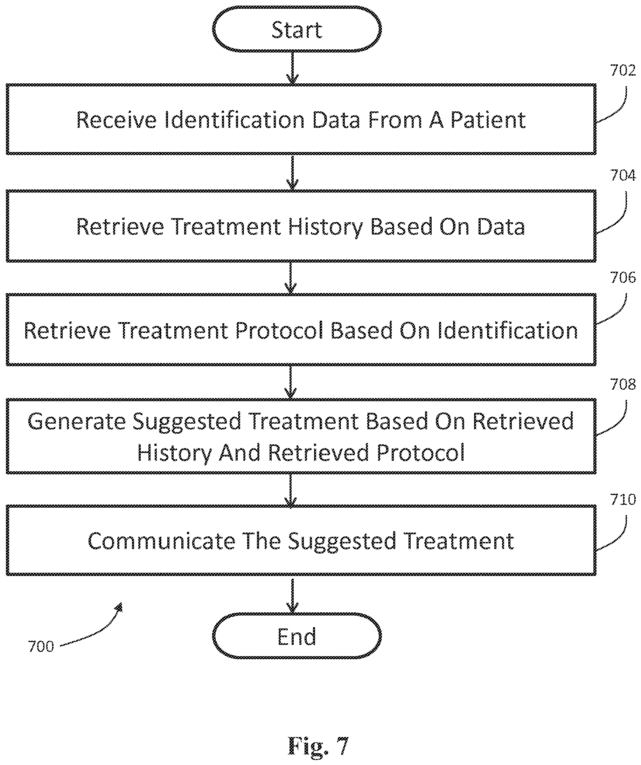

**FIG. 7** is a flow chart illustrating the steps of an example method 700 for facilitating phototherapeutic treatment. At step 702, phototherapeutic controller 106 receives identification data from a patient. At step 704, phototherapeutic controller 106 retrieves treatment history based on the identification data. At step 706, phototherapeutic controller 106 retrieves a treatment protocol based on the identification data. At step 708, phototherapeutic controller 106 generates a suggested treatment based on the retrieved treatment history and the retrieved treatment protocol. At step 710, phototherapeutic controller 106 communicates the suggested treatment to the patient.

In general, it will be apparent to one of ordinary skill in the art that at least some of the embodiments described herein may be implemented in many different embodiments of software, firmware, and/or hardware. The software and firmware code may be executed by a processor or any other similar computing device. The software code or specialized control hardware that may be used to implement embodiments is not limiting. For example, embodiments described herein may be implemented in computer software using any suitable computer software language type, using, for example, conventional or object-oriented techniques. Such software may be stored on any type of suitable computer-readable medium or media, such as, for example, a magnetic or optical storage medium. The operation and behavior of the embodiments may be described without specific reference to specific software code or specialized hardware components. The absence of such specific references is feasible, because it is clearly understood that artisans of ordinary skill would be able to design software and control hardware to implement the embodiments based on the present description with no more than reasonable effort and without undue experimentation.

Moreover, the processes associated with the present embodiments may be executed by programmable equipment, such as computers or computer systems and/or processors. Software that may cause programmable equipment to execute processes may be stored in any storage device, such as, for example, a computer system (nonvolatile) memory, an optical disk, magnetic tape, or magnetic disk. Furthermore, at least some of the processes may be programmed when the computer system is manufactured or stored on various types of computer-readable media.

It can also be appreciated that certain process aspects described herein may be performed using instructions stored on a computer-readable medium or media that direct a computer system to perform the process steps. A computer-readable medium may include, for example, memory devices such as diskettes, compact discs (CDs), digital versatile discs (DVDs), optical disk drives, or hard disk drives. A computer-readable medium may also include memory storage that is physical, virtual, permanent, temporary, semipermanent, and/or semitemporary.

A "computer," "computer system," "host," "server," or "processor" may be, for example and without limitation, a processor, microcomputer, minicomputer, server, mainframe, laptop, personal data assistant (PDA), wireless e-mail device, cellular phone, pager, processor, fax machine, scanner, or any other programmable device configured to transmit and/or receive data over a network. Computer systems and computer-based devices disclosed herein may include memory for storing certain software modules used in obtaining, processing, and communicating information. It can be appreciated that such memory may be internal or external with respect to operation of the disclosed embodiments. The memory may also include any means for storing software, including a hard disk, an optical disk, floppy disk, ROM (read only memory), RAM (random access memory), PROM (programmable ROM), EEPROM (electrically erasable PROM) and/or other computer-readable media.

In various embodiments disclosed herein, a single component may be replaced by multiple components and multiple components may be replaced by a single component to perform a given function or functions. Except where such substitution would not be operative, such substitution is within the intended scope of the embodiments. Any servers described herein, for example, may be replaced by a "server farm" or other grouping of networked servers (such as server blades) that are located and configured for cooperative functions. It can be appreciated that a server farm may serve to distribute workload between/among individual components of the farm and may expedite computing processes by harnessing the collective and cooperative power of multiple servers. Such server farms may employ load-balancing software that accomplishes tasks such as, for example, tracking demand for processing power from different machines, prioritizing and scheduling tasks based on network demand and/or providing backup contingency in the event of component failure or reduction in operability.

The computer systems may comprise one or more processors in communication with memory (e.g., RAM or ROM) via one or more data buses. The data buses may carry electrical signals between the processor(s) and the memory. The processor and the memory may comprise electrical circuits that conduct electrical current. Charge states of various components of the circuits, such as solid state transistors of the processor(s) and/or memory circuit(s), may change during operation of the circuits.

While various embodiments have been described herein, it should be apparent that various modifications, alterations, and adaptations to those embodiments may occur to persons skilled in the art with attainment of at least some of the advantages. The disclosed embodiments are therefore intended to include all such modifications, alterations, and adaptations without departing from the scope of the embodiments as set forth herein.

## Claims

1. A phototherapeutic system comprising a phototherapeutic device for facilitating phototherapeutic treatment, the system comprising:
an input device configured to receive input from a user, wherein the user is a patient or a medical professional;
a display device configured to communicate information to the user; and
a controller configured to:
receive patient identification data from the input device;
retrieve treatment history for the patient based on the patient identification data, wherein the treatment history comprises patient exposure history;
retrieve a treatment protocol based on the patient exposure history, wherein the treatment protocol comprises at least one treatment rule to decrease patient exposure if a treatment was missed; and
communicate the suggested current treatment to the display device.

2. The phototherapeutic system according to claim 1, further comprising a call device configured to receive a request from the patient during a phototherapeutic treatment.

3. The phototherapeutic system according to claim 2, wherein the controller is further configured to communicate a notification indicative of the request.

4. The phototherapeutic system according to claim 3, wherein the notification comprises one of a text message, an email message, a phone call, and an instant message.

5. The phototherapeutic system according to claim 3, wherein the controller configured to receive the patient identification data is further configured to:
receive biometric data; and
query a database to retrieve patient information corresponding to the received biometric data.

6. The phototherapeutic system according to any of the preceding claims, wherein the controller configured to receive the patient identification data is further configured to:
query a database to retrieve user information corresponding to the received data;
display the retrieved user information on the display device; and
wherein the patient information comprises at least one of a patient name and a patient photograph.

7. The phototherapeutic system according to any of the preceding claims, wherein the controller is further configured to:
receive confirmation to begin treatment; and
cause the phototherapeutic device to perform the suggested treatment.

8. The phototherapeutic system according to any of the preceding claims, wherein the controller is further configured to:
monitor a status of a current treatment being performed by the phototherapeutic device; and
communicate the status of the current treatment to the display device.

9. The phototherapeutic system according to any of the preceding claims, wherein the controller is further configured to store data representative of a current treatment in a database.

10. The phototherapeutic system according to any of the preceding claims, wherein the controller is further configured to communicate data representative of a calendar to the display device, wherein the calendar visually represent the retrieved treatment history.

## Patentansprüche

1. Phototherapeutisches System mit einer phototherapeutischen Vorrichtung zum Erleichtern einer phototherapeutischen Behandlung, wobei das System umfasst:
eine Eingabevorrichtung, die dazu eingerichtet ist, eine Eingabe von einem Benutzer zu empfangen, wobei der Benutzer ein Patient oder eine medizinische Fachkraft ist;
eine Anzeigevorrichtung, die dazu eingerichtet ist, Informationen an den Benutzer zu übermitteln; und
eine Steuerung, die eingerichtet ist, um:
Patientenidentifikationsdaten von der Eingabevorrichtung zu empfangen;
die Behandlungsvorgeschichte für den Patienten basierend auf den Patientenidentifikationsdaten abzurufen, wobei die Behandlungsvorgeschichte die Patienten-Bestrahlungsvorgeschichte umfasst;
ein Behandlungsprotokoll basierend auf der Patienten-Bestrahlungsvorgeschichte abzurufen, wobei das Behandlungsprotokoll mindestens eine Behandlungsregel umfasst, um die Patientenbestrahlung zu verringern, wenn eine Behandlung versäumt wurde; und
die vorgeschlagene aktuelle Behandlung an die Anzeigevorrichtung zu übermitteln.

2. Phototherapeutisches System nach Anspruch 1, ferner eine Anrufvorrichtung umfassend, die dazu eingerichtet ist, eine Anfrage von dem Patienten während einer phototherapeutischen Behandlung zu empfangen.

3. Phototherapeutisches System nach Anspruch 2, wobei die Steuerung ferner dazu eingerichtet ist, eine auf die Anfrage hinweisende Benachrichtigung zu übermitteln.

4. Phototherapeutisches System nach Anspruch 3, wobei die Benachrichtigung eines von einer Textnachricht, einer E-Mail-Nachricht, einem Telefonanruf und einer Sofortnachricht umfasst.

5. Therapeutisches System nach Anspruch 3, wobei die zum Empfangen der Patientenidentifikation eingerichtete Steuerung ferner eingerichtet ist, um:
biometrische Daten zu empfangen; und
eine Datenbank abzufragen, um den empfangenen biometrischen Daten entsprechende Patienteninformationen abzurufen.

6. Phototherapeutisches System nach einem der vorhergehenden Ansprüche, wobei die zum Empfangen der Patientenidentifikationsdaten eingerichtete Steuerung ferner eingerichtet ist, um:
eine Datenbank abzufragen, um den empfangenen Daten entsprechende Benutzerinformationen abzurufen;
die abgerufene Benutzerinformation auf der Anzeigevorrichtung anzuzeigen; und
wobei die Patienteninformationen mindestens eines von einem Patientennamen und einem Patientenfoto umfassen.

7. Phototherapeutisches System nach einem der vorhergehenden Ansprüche, wobei die Steuerung ferner eingerichtet ist, um:
eine Bestätigung zu empfangen, um die Behandlung zu beginnen; und
zu bewirken, dass die phototherapeutische Vorrichtung die vorgeschlagene Behandlung ausführt.

8. Phototherapeutisches System nach einem der vorhergehenden Ansprüche, wobei die Steuerung ferner eingerichtet ist, um:
einen Status einer aktuellen Behandlung zu überwachen, die durch die phototherapeutische Vorrichtung ausgeführt wird; und
den Status der aktuellen Behandlung an die Anzeigevorrichtung zu übermitteln.

9. Phototherapeutisches System nach einem der vorhergehenden Ansprüche, wobei die Steuerung ferner dazu eingerichtet ist, in einer Datenbank Daten zu speichern, die für eine aktuelle Behandlung repräsentativ sind.

10. Phototherapeutisches System nach einem der vorhergehenden Ansprüche, wobei die Steuerung ferner dazu eingerichtet ist, an die Anzeigevorrichtung Daten zu übermitteln, die für einen Kalender repräsentativ sind, wobei der Kalender die abgerufene Behandlungsvorgeschichte visuell darstellt.

## Revendications

1. Système photothérapeutique comprenant un dispositif photothérapeutique destiné à faciliter un traitement photothérapeutique, le système comprenant :
un dispositif d'entrée configuré pour recevoir une entrée d'un utilisateur, l'utilisateur étant un patient ou un professionnel de santé ;
un dispositif d'affichage configuré pour communiquer des informations à l'utilisateur ; et
une unité de commande configurée pour :
recevoir des données d'identification de patient à partir du dispositif d'entrée ;
récupérer un historique de traitement pour le patient sur la base des données d'identification de patient, l'historique de traitement comprenant l'historique d'exposition du patient ;
récupérer un protocole de traitement sur la base de l'historique d'exposition du patient, le protocole de traitement comprenant au moins une règle de traitement pour diminuer l'exposition du patient si un traitement a été manqué ; et
communiquer le traitement courant suggéré au dispositif d'affichage.

2. Système photothérapeutique selon la revendication 1, comprenant en outre un dispositif d'appel configuré pour recevoir une demande du patient lors d'un traitement photothérapeutique.

3. Système photothérapeutique selon la revendication 2, dans lequel l'unité de commande est en outre configurée pour communiquer une notification indicatrice de la demande.

4. Système photothérapeutique selon la revendication 3, dans lequel la notification comprend un parmi un message texte, un message électronique, un appel téléphonique et un message instantané.

5. Système photothérapeutique selon la revendication 3, dans lequel l'unité de commande configurée pour recevoir les données d'identification de patient est en outre configurée pour :
recevoir des données biométriques ; et
interroger une base de données pour récupérer des informations de patient correspondant aux données biométriques reçues.

6. Système photothérapeutique selon l'une quelconque des revendications précédentes, dans lequel l'unité de commande configurée pour recevoir les données d'identification de patient est en outre configurée pour :
interroger une base de données pour récupérer des informations d'utilisateur correspondant aux données reçues ;
afficher les informations d'utilisateur récupérées sur le dispositif d'affichage ; et
les informations de patient comprenant au moins un parmi un nom de patient et une photographie de patient.

7. Système photothérapeutique selon l'une quelconque des revendications précédentes, dans lequel l'unité de commande est en outre configurée pour :
recevoir une confirmation de commencement d'un traitement ; et
amener le dispositif photothérapeutique à réaliser le traitement suggéré.

8. Système photothérapeutique selon l'une quelconque des revendications précédentes, dans lequel l'unité de commande est en outre configurée pour :
surveiller un état d'un traitement courant qui est réalisé par le dispositif photothérapeutique ; et
communiquer l'état du traitement courant au dispositif d'affichage.

9. Système photothérapeutique selon l'une quelconque des revendications précédentes, dans lequel l'unité de commande est en outre configurée pour stocker des données représentatives d'un traitement courant dans une base de données.

10. Système photothérapeutique selon l'une quelconque des revendications précédentes, dans lequel l'unité de commande est en outre configurée pour communiquer des données représentatives d'un calendrier au dispositif d'affichage, le calendrier représentant visuellement l'historique de traitement récupéré.
